# FASCICULE DE BREVET EUROPEEN

(11) **EP 2 054 666 B1**
(45) Date de publication et mention de la délivrance du brevet: **25.04.2018**
(21) Numéro de dépôt: 07823427.5
(22) Date de dépôt: 16.08.2007
(51) Int. Cl.: F23D 3/02, F23D 3/08, F23C 13/00, A61L 9/03

(54) **BRULEUR A COMBUSTION CATALYTIQUE PRESENTANT UN EMBOUT PARTICULIER ET FLACON EQUIPE D'UN TEL BRULEUR**
KATALYTISCHER BRENNER MIT SPEZIALSPITZE UND MIT EINEM SOLCHEN BRENNER AUSGESTATTETE FLASCHE
CATALYTIC BURNER WITH A SPECIAL TIP AND FLASK EQUIPPED OF SUCH A BURNER

(30) Priorité: 23.08.2006 FR 0607473
(43) Date de publication de la demande: 06.05.2009
(73) Titulaire: PRODUITS BERGER, 27520 Bourgtheroulde-Infreville (FR)
(72) Inventeur: GOMEZ, Corinne, F-27400 Incarville (FR); DESDOITS, Michel, 27520 Bourgtheroulde (FR); LENTZ, Philippe, 78240 Chambourcy (FR)
(74) Mandataire: Novagraaf Technologies
(86) Numéro de dépôt international: PCT/FR2007/001376
(87) Numéro de publication internationale: WO 2008/023112

(56) Documents cités:
- FR-A1- 2 610 390
- FR-A1- 2 770 282
- FR-A1- 2 779 509
- FR-A1- 2 856 775

## Description

La présente invention concerne, de façon générale, le domaine de la combustion catalytique, et plus précisément des brûleurs à combustion catalytique en matière poreuse.

En particulier, ces brûleurs sont utilisés pour la diffusion de parfum, pour la destruction de molécules odorantes ou non, et/ou pour l'assainissement de l'air.

Le parfum se trouve dans un solvant, qui est souvent un alcool, et par exemple l'alcool isopropylique.

Des diffuseurs de parfum comprenant ces brûleurs doivent assurer une bonne diffusion du parfum et du solvant, qui peut avoir une activité bactéricide.

Plus particulièrement, l'invention concerne, selon un premier de ses aspects, un brûleur à combustion catalytique comprenant un embout en matière poreuse présentant une paroi frontale supérieure et une paroi périphérique latérale délimitant ensemble une cavité s'étendant le long d'un axe principal et débouchant par une ouverture inférieure. Cet embout est destiné à venir coiffer une extrémité supérieure d'une mèche engagée dans la cavité suivant une première direction et propre à imbiber l'embout d'une composition combustible. Cet embout présente en outre une face supérieure formée d'une zone centrale et d'une zone périphérique séparées l'une de l'autre par une gorge annulaire creusée dans l'embout suivant une deuxième direction inverse de la première. La paroi périphérique présente une face latérale externe dont une partie au moins est dopée par un catalyseur, et la zone centrale de la face supérieure est exempte de catalyseur.

La zone centrale forme une zone de diffusion et la zone périphérique forme une zone de catalyse.

Un tel brûleur est décrit dans le document FR 2 610 390.

Toutefois, dans ce type de brûleur utilisé aujourd'hui, la cartographie des températures des zones de catalyse et de diffusion n'est pas bien maîtrisée et n'est donc pas entièrement satisfaisante.

En particulier, la température de la zone de diffusion n'est pas constante et peut atteindre une température de 250 degrés Celsius.

Une telle température de la zone de diffusion peut entraîner l'altération de la qualité olfactive du parfum diffusé à cause de la destruction des molécules légères. L'efficacité bactéricide peut également être altérée à cause d'une diffusion en solvant, tel que l'alcool isopropylique, moins importante.

La température idéale pour la zone de diffusion est d'environ 200 degrés Celsius, soit entre 160 et 230 degrés Celsius.

Cette température ne doit pas non plus être inférieure. Une température trop faible de la zone de diffusion peut être préjudiciable en ce que la diffusion des molécules de poids moléculaire élevé, telles que certaines molécules de parfum ou des pyréthrinoïdes, n'est plus correctement assurée.

En outre, une température trop faible de la zone de diffusion est préjudiciable en ce que cela peut entraîner un refroidissement de la zone de catalyse qui n'est plus alors à une température permettant un fonctionnement optimal du brûleur. De manière avantageuse, la température de la zone de catalyse est comprise entre 450 et 540 degrés Celsius, pour permettre non seulement une bonne combustion, mais aussi la destruction de molécules de l'atmosphère, odorantes ou non, qui viendraient circuler à proximité de cette zone.

Dans ce contexte, la présente invention a pour but de proposer un brûleur exempt de l'une au moins des limitations précédemment évoquées.

A cette fin, le brûleur de l'invention, par ailleurs conforme à la définition générique qu'en donne le préambule ci-dessus, est essentiellement caractérisé en ce que la gorge est délimitée par des faces latérales inclinées par rapport à l'axe principal et divergeant l'une de l'autre suivant la première direction, et en ce que l'embout inclut des premier et deuxième éléments distincts, qui sont respectivement une tête formée de l'embout exempt de zone centrale et un insert formant ladite zone centrale. L'invention présente l'avantage de présenter une cartographie de températures satisfaisante et améliorée par rapport aux brûleurs à combustion catalytique connus.

Dans un mode de réalisation préféré de l'invention, les faces latérales de la gorge sont séparées d'un angle alpha compris entre 30 et 40° (degrés).

La gorge annulaire présente préférentiellement une profondeur comprise entre 30 et 55%, de préférence entre 40 et 50%, de la hauteur de l'embout.

Tout préférentiellement, les premier et deuxième éléments présentent au moins une surface de contact l'un avec l'autre.

Selon une version particulière de l'invention, les faces latérales de la gorge, de préférence une face latérale centrale de la gorge, et/ou une surface frontale de la zone centrale comportent des évidements.

Tout particulièrement, les évidements occupent par exemple une surface au moins égale à 5%, de préférence comprise entre 10 et 60%, des faces latérales de la gorge, de préférence de la face latérale centrale de la gorge, et/ou d'une surface frontale de la zone centrale.

La zone périphérique de l'embout présente avantageusement une surface frontale exempte de catalyseur.

Selon un mode de réalisation particulier, l'embout comporte au moins un canal ouvert faisant communiquer la cavité avec l'atmosphère.

De préférence, la cavité de l'embout se prolonge au niveau de l'ouverture inférieure par une cavité d'un manchon en matière poreuse et adapté à enserrer la mèche.

La face latérale externe peut être inclinée sur au moins une partie de sa hauteur par rapport à l'axe principal, l'inclinaison étant telle que le diamètre de l'embout augmente dans la première direction.

Selon une version toute particulière de l'invention, la paroi périphérique latérale présente un bord d'appui adapté à être supporté par un dispositif de support, ledit bord d'appui comportant au moins une zone creuse propre à définir un passage d'air à la périphérie du brûleur.

L'invention concerne également un dispositif de combustion catalytique comprenant un récipient adapté à recevoir une composition combustible, ledit récipient comportant un goulot auquel est fixée une embase comportant un trou faisant communiquer l'intérieur du récipient avec l'atmosphère, et ladite embase recevant un brûleur selon l'invention.

D'autres caractéristiques et avantages de l'invention ressortiront clairement de la description détaillée qui en est faite ci-après, à titre indicatif et nullement limitatif, en référence aux dessins annexés dans lesquels :
- la figure 1 représente schématiquement une coupe transversale d'un brûleur selon l'invention ;
- la figure 2 représente schématiquement une coupe transversale d'un autre brûleur selon l'invention ;
- la figure 3 représente une vue en perspective d'un brûleur selon l'invention ;
- la figure 4A représente une vue schématique de dessus d'un insert selon l'invention et la figure 4B représente des dessins en vue en perspective de cet insert ;
- la figure 5 représente schématiquement une coupe transversale d'une tête selon l'invention, comportant un manchon en matière poreuse.
Les caractéristiques techniques communes à plusieurs figures sont désignées chacune par la même référence numérique dans les figures concernées.

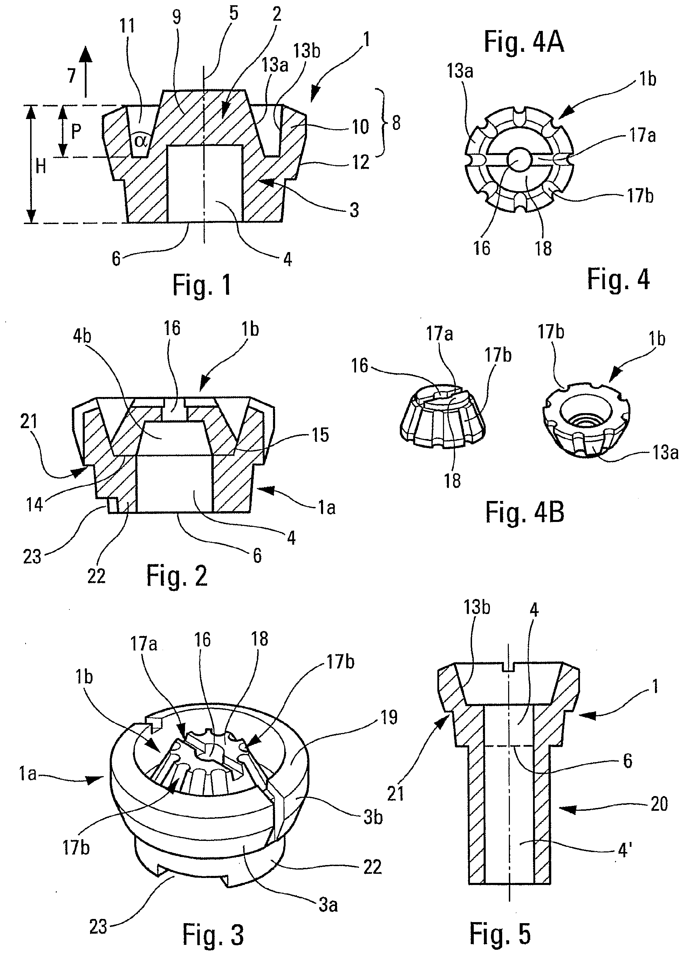

Le brûleur à combustion catalytique représenté à la figure 1 comprend un embout 1 en matière poreuse.

L'embout 1 présente une paroi frontale supérieure 2 et une paroi périphérique latérale 3, qui délimitent ensemble une cavité 4 s'étendant le long d'un axe principal 5 et débouchant par une ouverture inférieure 6.

Le diamètre de cette cavité 4 est avantageusement compris entre 5 et 9 millimètres, de préférence entre 6 et 8,5 millimètres.

L'embout 1 est destiné à venir coiffer une extrémité supérieure d'une mèche engagée dans la cavité 4 suivant une première direction 7 et propre à imbiber l'embout 1 d'une composition combustible.

Cet embout 1 présente en outre une face supérieure 8 formée d'une zone centrale 9 et d'une zone périphérique 10 séparées l'une de l'autre par une gorge annulaire 11 creusée dans l'embout 1 suivant une deuxième direction inverse de la première direction 7.

La paroi périphérique 3 présente une face latérale externe 12 dont une partie au moins est dopée par un catalyseur. Selon un mode de réalisation préférentiel de l'invention, au moins le quart inférieur de la face latérale 12 est exempt de catalyseur. Tout préférentiellement, la partie de la face latérale 12 exempte de catalyseur correspond à une partie inférieure de cette face 12, présentant une hauteur comprise entre le tiers et la moitié de la hauteur de la face latérale 12.

De manière particulièrement avantageuse, la zone centrale 9 de la face supérieure 8 est exempte de catalyseur, ce qui permet de limiter la température dans cette zone au niveau de laquelle les molécules de la composition à diffuser ne sont ainsi pas détruites.

La gorge 11 est délimitée par des faces latérales 13a et 13b inclinées par rapport à l'axe principal 5 et divergeant l'une de l'autre suivant la première direction 7.

De préférence, les faces latérales 13a et 13b de la gorge 11 sont séparées d'un angle α (alpha) compris entre 30 et 40 degrés.

La gorge annulaire 11 est délimitée plus précisément par une face latérale centrale 13a et une face latérale périphérique 13b. Une configuration avantageuse du brûleur est celle dans laquelle l'inclinaison de la face latérale centrale 13a et de la face latérale périphérique 13b par rapport à l'axe principal 5 est respectivement de 19 et 16 degrés, à plus ou moins 2 ou 3 degrés.

La gorge annulaire 11 présente avantageusement une profondeur P comprise entre 30 et 55%, de préférence entre 40 et 50%, de la hauteur H de l'embout 1.

Par exemple, l'embout 1 présente une hauteur H comprise entre 10 et 15 millimètres et la gorge présente une profondeur P comprise entre 4,5 et 7,5 millimètres.

Dans la figure 2, le brûleur présente un embout 1 incluant des premier et deuxième éléments distincts la et 1b, qui sont respectivement une tête la formée de l'embout 1 exempt de zone centrale et un insert 1b formant la zone centrale.

L'insert 1b est en matière poreuse. De préférence, la tête la et l'insert 1b sont fabriqués à partir d'une même composition. Toutefois, dans la mise en oeuvre de l'invention, on peut choisir deux matériaux poreux différents pour la tête la et l'insert 1b.

Les premier et deuxième éléments 1a et 1b présentent préférentiellement au moins une surface 14, 15 de contact l'un avec l'autre. Cette surface de contact permet le passage direct des fluides des pores du premier élément à ceux du deuxième élément.

Par exemple, à la figure 2 sont représentées deux surfaces de contact entre les premier et deuxième éléments, une première surface 14 de contact horizontale et une deuxième surface 15 de contact entre les faces latérales centrale 13a et périphérique 13b de la gorge 11.

Dans cette figure 2, la cavité 4 est ménagée dans le premier élément la et dans le deuxième élément 1b. Dans un autre mode de réalisation de l'invention, le deuxième élément 1b n'inclut pas une partie 4b de la cavité 4.

L'embout 1 peut comporter au moins un canal ouvert 16 faisant communiquer la cavité 4 avec l'atmosphère. Un brûleur avec un tel canal 16 est décrit dans le brevet FR 2 779 509.

La présence du canal 16 permet de faciliter encore le refroidissement de la zone centrale 9, et donc l'obtention d'une température idéale dans cette zone 9 et aussi dans la zone périphérique 10.

En outre, le canal ouvert 16 permet d'éviter que l'extrémité de la mèche ne carbonise dans le brûleur.

Afin d'augmenter la surface d'échange avec l'air atmosphérique, des évidements 17a et 17b peuvent être prévus au niveau de l'embout 1. Ils peuvent être situés au niveau des faces latérales 13a et 13b de la gorge 11 et/ou d'une surface frontale 18 de la zone centrale 9.

De préférence, la face latérale centrale 13a de la gorge 11 inclut des évidements, dans le but d'entraîner une baisse de la température de la zone centrale 9 par contact avec l'air atmosphérique.

On peut prévoir toute forme d'évidement telle que strie, poinçon, rainure, cercle, spirale, et dans toute direction.

La surface frontale 18 de la zone centrale 9 peut aussi inclure au moins un évidement. Par exemple, elle inclut un évidement 17a tel que représenté à la figure 3, qui peut servir à positionner un étrier pour le maintien éventuel du brûleur sur un support.

De manière particulièrement avantageuse, les évidements occupent une surface au moins égale à 5%, généralement comprise entre 5 et 80%, avantageusement entre 10 et 60%, et de préférence entre 25 et 45%, d'une surface totale représentée par la face latérale centrale 13a de la gorge 11, éventuellement la face latérale périphérique 13b de la gorge 11, et éventuellement la surface frontale 18 de la zone centrale 9.

A titre d'exemple, un insert 1b comprenant des évidements particuliers est représenté à la figure 4. L'insert 1b est de forme essentiellement tronconique.

Les évidements particuliers sont des rainures latérales 17b réalisées sur le pourtour de l'insert 1b représentant la face latérale centrale 13a de la gorge 11. Un évidement sous la forme d'une rainure frontale 17a est également présent sur le dessus de l'insert 1b représentant la surface frontale 18 de la zone centrale 9, et peut être utile au positionnement d'un étrier.

Dans ce mode particulier de réalisation, les rainures latérales 17b sont au nombre de 8. Elles sont creusées dans un insert 1b de l'ordre de 12 millimètres de plus grand diamètre et d'environ 6 millimètres de hauteur, en décrivant un arc de cercle de rayon égal à 0,75 millimètres. Un angle de 45 degrés sépare chaque rainure 17b de chaque rainure 17b adjacente.

Dans les figures 3 et 4, l'embout 1 comporte également un canal ouvert 16.

De manière particulièrement avantageuse, la zone périphérique 10 présente une surface frontale 19 exempte de catalyseur.

Le brûleur de la figure 5 comporte un manchon 20 en matière poreuse et adapté à enserrer la mèche. Ce manchon 20 comporte une cavité 4' se trouvant dans le prolongement de la cavité 4 de l'embout 1. Un tel manchon est décrit dans le brevet FR 2 856 776.

Le manchon 20 est constitué d'un matériau identique ou différent de chaque matériau qui constitue l'embout 1. Ainsi, la tête la, l'insert 1b et le manchon 20 sont préférentiellement fabriqués à partir d'une même composition mais peuvent aussi être fabriqués à partir de deux ou trois compositions différentes.

De manière avantageuse, le manchon 20 recouvre au moins 5%, de préférence entre 5 et 40%, et plus préférentiellement entre 7 et 20%, de la longueur de la mèche.

La cavité 4' est adaptée à recevoir la mèche. Le diamètre de cette cavité 4' est avantageusement compris entre 5 et 9 millimètres, de préférence entre 6 et 8,5 millimètres.

La présence d'un manchon 20 est avantageuse pour réguler l'apport de composition combustible à l'embout 1, en particulier quel que soit le niveau de composition combustible dans lequel trempe la mèche.

L'apport de composition combustible à l'embout d'un brûleur selon l'invention doit être suffisant pour permettre un apport suffisant des molécules de parfum et de combustible, donc une bonne réaction de combustion au niveau de la zone catalytique, et également un refroidissement de la zone de diffusion. Mais un apport trop important de composition combustible à l'embout peut entraîner un engorgement et le non fonctionnement du brûleur.

Selon une version particulière de l'invention, la face latérale externe 12 de l'embout 1 est inclinée sur au moins une partie de sa hauteur par rapport à l'axe principal 5, l'inclinaison étant telle que le diamètre de l'embout 1 augmente dans la première direction 7.

L'inclinaison est en particulier avantageuse en ce qu'elle permet d'augmenter le temps de contact de l'air atmosphérique avec la zone de catalyse et ainsi de faciliter la destruction de molécules présentes dans cet air et responsables par exemple de mauvaises odeurs.

Une telle configuration est décrite dans le brevet FR 2 856 775.

De préférence, la face latérale externe 12 comporte au moins un épaulement 21.

Un épaulement 21 peut par exemple séparer la face latérale externe 12 en une portion haute dopée par le catalyseur et une portion basse exempte de catalyseur. La présence de cet épaulement est donc avantageuse en particulier au moment de la fabrication du brûleur pour délimiter la portion sur laquelle doit être déposé du catalyseur.

De manière particulièrement avantageuse, la face latérale externe 12 de l'embout 1 présente la configuration telle que représentée dans les figures. Dans cette configuration avantageuse, la face latérale externe 12 comporte un épaulement 21. Au-dessus de cet épaulement 21, la face latérale externe 12 comprend une paroi inclinée 3a en-dessous d'une paroi droite 3b, elle-même en-dessous de la surface frontale 19 de la zone périphérique qui peut être légèrement inclinée ou horizontale.

Ainsi, on conserve une paroi inclinée et les avantages y afférents et on inclut une paroi droite qui peut présenter, selon la technique de dépôt utilisée, l'avantage de faciliter le dépôt de catalyseur.

En outre, cette configuration particulière est avantageuse pour la fabrication du brûleur en ce qu'elle en facilite le démoulage.

La surface frontale 19 de la zone périphérique 10 est avantageusement exempte de catalyseur. L'air atmosphérique, en fonction des mouvements de convection qui se créent naturellement autour de l'embout 1 du brûleur, n'entre normalement que très peu en contact avec la surface frontale 19 de la zone périphérique 10. Le catalyseur éventuellement déposé sur cette surface 19 est donc peu utile à l'action du brûleur, et ce dépôt représente plutôt une perte de catalyseur et donc n'est pas économique. En outre, la présence de catalyseur sur cette surface 19 peut entraîner un réchauffement non souhaitable de la zone centrale 9 de diffusion.

On préfère limiter le dépôt de catalyseur au niveau de la paroi droite 3b de la face latérale externe 12 et d'une partie au moins de la paroi inclinée 3a de cette face 12.

L'invention présente l'avantage de proposer un brûleur dans lequel on maîtrise mieux la cartographie des températures des zones de diffusion et de catalyse. L'invention permet l'obtention de très bonnes performances de diffusion. L'efficacité bactéricide est au moins égale à celle du brûleur décrit dans le brevet FR 2 779 509, et la qualité olfactive est améliorée. Les molécules, odorantes ou non, présentes dans l'air atmosphérique sont mieux détruites et les molécules de parfum de la composition combustible sont mieux diffusées.

En outre, comme représenté aux figures 2 et 3, la paroi périphérique latérale 3 présente un bord 22 d'appui adapté à être supporté par un dispositif de support. Ce bord 22 d'appui comporte de manière avantageuse au moins une zone creuse 23 propre à définir un passage d'air à la périphérie du brûleur.

Chaque zone creuse 23 se présente sous toute forme de découpe, par exemple sous la forme de créneaux ou de cannelures, et dont les angles éventuels sont droits ou arrondis. Les zones creuses 23 représentées aux figures 2 et 3 à titre d'exemple sont des créneaux aux angles droits.

L'embout 1 du brûleur présente une surface totale de paroi périphérique latérale 3 dans un plan de section passant par le bord 22 d'appui. Dans un mode de réalisation préféré de l'invention, au moins une zone creuse 23 vue en section selon ce plan de section présente une surface totale de passage d'air au moins égale à 10% de la surface totale de paroi périphérique latérale 3.

La ou les zones creuses 23 occupent par exemple un angle total d'au moins 35 degrés, de préférence un angle total compris entre 35 et 290 degrés, tout préférentiellement entre 70 et 255 degrés, ou encore plus préférentiellement entre 100 et 220 degrés, à la périphérie du brûleur.

Selon un exemple particulier, le brûleur peut inclure trois zones creuses 23 réparties régulièrement à la périphérie du brûleur au niveau de son bord 22 d'appui. Chaque évent ou zone creuse 23 occupe un angle de 60 degrés à la périphérie du brûleur. Les trois zones creuses 23 sont séparées chacune l'une de l'autre de 60 degrés.

Selon une version particulière de l'invention, le dispositif de support adapté à recevoir un brûleur selon l'invention inclut un récipient comprenant un goulot formant une ouverture de passage de brûleur.

De plus, de manière avantageuse, le dispositif de support inclut au moins une pièce distincte du récipient. L'une au moins de ces pièces est positionnée dans le goulot. Le goulot et/ou l'une au moins de ces pièces comportent l'ouverture de passage de brûleur.

Selon l'invention, le dispositif de support peut inclure par exemple un récipient, un goulot, une embase et/ou un moyen de support.

Le récipient est susceptible de contenir une composition combustible propre à imbiber le brûleur par l'intermédiaire d'une mèche.

Selon une version particulièrement avantageuse de l'invention, le dispositif de support inclut un moyen de support métallique adapté à recevoir le brûleur, installé sur une embase métallique dans le goulot du récipient.

La zone creuse du brûleur définit alors un passage d'air entre un volume intérieur du récipient et un volume extérieur à l'ensemble brûleur-dispositif de support.

L'invention présente l'avantage de permettre la mise en oeuvre d'un ensemble de combustion catalytique avec un ou plusieurs évents qui permettent un passage d'air entre le brûleur et un dispositif de support quelconque.

Une ventilation naturelle est ainsi créée, permettant d'améliorer en fonctionnement l'évacuation des gaz accumulés dans le récipient contenant la composition combustible.

En outre, l'absence de contact entre le dispositif de support et le brûleur au niveau de la zone creuse permet un passage d'air entre les deux éléments et donc leur refroidissement à l'endroit et autour de la zone creuse, ce qui participe au respect de la cartographie des températures au sein du brûleur.

La présence de zones creuses 23 sur un brûleur comportant un manchon 20 est particulièrement avantageuse. En effet, la présence de ce manchon 20 entraîne une conduction de chaleur plus importante en fonctionnement dans un récipient. Les zones creuses 23 permettent une circulation d'air limitant cette conduction de chaleur vers le récipient et favorisant l'évacuation des gaz accumulés dans ce récipient.

L'invention concerne également un dispositif de combustion catalytique comprenant un récipient adapté à recevoir une composition combustible. Le récipient comporte un goulot auquel est fixée une embase comportant un trou faisant communiquer l'intérieur du récipient avec l'atmosphère. Un tel dispositif est connu du document FR 2 779 509. L'embase du dispositif est adaptée à recevoir un brûleur selon l'invention.

## Revendications

1. Brûleur à combustion catalytique comprenant Un embout (1) en matière poreuse présentant une paroi frontale supérieure (2) et une paroi périphérique latérale (3) délimitant ensemble une cavité (4) s'étendant le long d'un axe principal (5) et débouchant par une ouverture inférieure (6), cet embout (1) étant destine à venir coiffer une extrémité supérieure d'une mèche engagée dans la cavité (4) suivant une première direction (7) et propre imbiber l'embout (1) d'une composition combustible, cet embout (1) présentant en outre une face supérieure (8) formée d'une zone centrale (9) et d'une zone périphérique (10) séparées l'une de l'autre par une gorge annulaire (11) creusée dans l'embout (1) suivant une deuxième direction inverse de la première (7), la paroi périphérique (3) présentant une face latérale externe (12) dont une partie au moins est dopée par un catalyseur, et la zone centrale (9) de la face supérieure (8) étant exempte de catalyseur,
**caractérisé en ce que** la gorge (11) est délimitée par des faces latérales (13a, 13b) inclinées par rapport à l'axe principal (5) et divergeant l'une de l'autre suivant la première direction (7), et **en ce que** l'embout (1) inclut des premier et deuxième éléments (1a, 1b) distincts, qui sont respectivement une tête (1a) formée de l'embout (1) exempt de zone centrale (9) et un insert (1b) formant ladite zone centrale (9).

2. Brûleur selon la revendication 1, dans lequel les faces latérales (13a, 13b) de la gorge (11) sont séparées d'un angle alpha (a) compris entre 30 et 40 degrés.

3. Brûleurs selon la revendication 1 ou 2, dans lequel la gorge annulaire (11) présente une profondeur (P) comprise entre 30 et 55%, de préférence entre 40 et 50%, de la hauteur (H) de l'embout (1).

4. Brûleur selon l'une quelconque des revendications 1 à 3, dans lequel les premier et deuxième éléments (1a, 1b) présentent au moins une surface de contact (14, 15) l'un avec l'autre.

5. Brûleur selon l'une quelconque des revendications 1 à 4, dans lequel les faces latérales (13a, 13b) de la gorge (11), de préférence une face latérale centrale (13a) de la gorge (11), et/ou une surface frontale (19) de la zone centrale (9) comportent des évidements (17a, 17b) .

6. Brûleurs selon la revendication 5, dans lequel les évidements (17a, 17b) occupent une surface au moins égale à 5%, de préférence comprise entre 10 et 60%, des faces latérales (13a, 13b) de la gorge (11), de préférence de la face latérale centrale (13a) de la gorge (11), et/ou d'une surface frontale (19) de la zone centrale (9).

7. Brûleur selon l'une quelconque des revendications 1 à 6, dans lequel la zone périphérique (10) présente une surface frontale (19) exempte de catalyseur.

8. Brûleur selon l'une quelconque des revendications 1 à 7, dans lequel l'embout (1) comporte au moins un canal ouvert (16) faisant communiquer la cavité (4, 4b) avec 1' atmosphère.

9. Brûleur selon l'une quelconque des revendications 1 à 8, dans lequel la cavité (4) de l'embout (1) se prolonge au niveau de l'ouverture inférieure (6) par une cavité (4') d'un manchon (20) en matière poreuse et adapté à enserrer la mèche.

10. Brûleur selon l'une quelconque des revendications 1 à 9, dans lequel la face latérale externe (12) est inclinée sur au moins une partie de sa hauteur par rapport a l'axe principal (5), l'inclinaison étant telle que le diamètre de l'embout (1) augmente dans la première direction (7).

11. Brûleur selon l'une quelconque des revendications 1 à 10, dans lequel la paroi périphérique latérale (3) présente un bord (22) d'appui adapté à être supporté par un dispositif de support, ledit bord (22) d'appui comportant au moins une zone creuse (23) propre à définir un passage d'air à la périphérie du brûleur.

12. Dispositif de combustion catalytique comprenant un récipient adapté à recevoir une composition combustible, ledit récipient comportant un goulot auquel est fixée une embase comportant un trou faisant communiquer l'intérieur du récipient avec l'atmosphère, et ladite embase recevant un brûleur selon l'une quelconque des revendications 1 à 11.

## Patentansprüche

1. Brenner mit katalytischer Verbrennung, umfassend einen Stutzen (1) aus porösem Material, aufweisend eine obere frontale Wand (2) und eine laterale periphere Wand (3), die zusammen einen Hohlraum (4) begrenzen, der sich entlang einer Hauptachse (5) erstreckt und durch eine untere Öffnung (6) mündet, wobei dieser Stutzen (1) ausgelegt ist, um ein oberes Ende eines Dochts abzudecken, der in den Hohlraum (4) gemäß einer ersten Richtung (7) eingreift und geeignet ist, um den Stutzen (1) mit einer brennbaren Zusammensetzung zu tränken, wobei dieser Stutzen (1) außerdem eine obere Seite (8) aufweist, die aus einem zentralen Bereich (9) und einem peripheren Bereich (10) gebildet ist, die voneinander durch eine ringförmige Furche (11) getrennt sind, die in den Stutzen (1) gemäß einer zweiten Richtung, entgegen der ersten (7), eingearbeitet ist, wobei die periphere Wand (3) eine äußere laterale Seite (12) aufweist, von der mindestens ein Teil mit einem Katalysator versetzt ist und der zentrale Bereich (9) der oberen Seite (8) ohne Katalysator ist,
**dadurch gekennzeichnet, dass** die Furche (11) durch laterale Seiten (13a, 13b) begrenzt ist, die mit Bezug auf die Hauptachse (5) geneigt sind und beide voneinander gemäß der ersten Richtung (7) divergieren, und
dadurch, dass der Stutzen (1) verschiedene erste und zweite Elemente (1a, 1b) einschließt, die jeweils ein Kopf (1a) sind, der vom Stutzen (1) gebildet ist, der ohne zentralen Bereich (9) ist, und ein Einsatzstück (1b), das den zentralen Bereich (9) bildet.

2. Brenner nach Anspruch 1, wobei die lateralen Seiten (13a, 13b) der Furche (11) um einen Winkel α (alpha) getrennt sind, der zwischen 30 und 40 Grad liegt.

3. Brenner nach Anspruch 1 oder 2, wobei die ringförmige Furche (11) eine Tiefe (P) aufweist, die zwischen 30 und 55 %, vorzugsweise zwischen 40 und 50 %, der Höhe (H) des Stutzens (1) liegt.

4. Brenner nach einem der Ansprüche 1 bis 3, wobei das erste und das zweite Element (1a, 1b) mindestens eine Kontaktoberfläche (14, 15) miteinander aufweisen.

5. Brenner nach einem der Ansprüche 1 bis 4, wobei die lateralen Seiten (13a, 13b) der Furche (11), vorzugsweise eine zentrale laterale Seite (13a) der Furche (11), und/oder eine frontale Oberfläche (19) des zentralen Bereichs (9) mit Aussparungen (17a, 17b) ausgestattet sind.

6. Brenner nach Anspruch 5, wobei die Aussparungen (17a, 17b) eine Oberfläche von mindestens gleich 5 %, vorzugsweise im Bereich zwischen 10 und 60 %, der lateralen Seiten (13a, 13b) der Furche (11), vorzugsweise der zentralen lateralen Seite (13a) der Furche (11), und/oder einer frontalen Oberfläche (19) des zentralen Bereichs (9) einnimmt.

7. Brenner nach einem der Ansprüche 1 bis 6, wobei der periphere Bereich (10) eine frontale Oberfläche (19), die ohne Katalysator ist, aufweist.

8. Brenner nach einem der Ansprüche 1 bis 7, wobei der Stutzen (1) mit mindestens einem offenen Kanal (16) ausgestattet ist, der den Hohlraum (4, 4b) mit der Atmosphäre verbindet.

9. Brenner nach einem der Ansprüche 1 bis 8, wobei sich der Hohlraum (4) des Stutzens (1) auf der Ebene der unteren Öffnung (6) durch einen Hohlraum (4') einer Muffe (20) aus porösem Material verlängert und angepasst ist, um den Docht zu umschließen.

10. Brenner nach einem der Ansprüche 1 bis 9, wobei die externe laterale Seite (12) auf mindestens einem Teil ihrer Höhe mit Bezug auf die Hauptachse (5) geneigt ist, wobei die Neigung derart ist, dass sich der Durchmesser des Stutzens (1) in der ersten Richtung (7) erhöht.

11. Brenner nach einem der Ansprüche 1 bis 10, wobei die laterale periphere Seite (3) einen Auflagerand (22) aufweist, der angepasst ist, um von einer Tragevorrichtung getragen zu werden, wobei der Auflagerand (22) mindestens einen hohlen Bereich (23) umfasst, der geeignet ist, um einen Durchgang von Luft an die Peripherie des Brenners zu definieren.

12. Vorrichtung zur katalytischen Verbrennung, umfassend einen Behälter, der angepasst ist, um eine brennbare Zusammensetzung aufzunehmen, wobei der Behälter einen Hals umfasst, an den ein Sockel befestigt ist, ausgestattet mit einem Loch, das das Innere des Behälters mit der Atmosphäre verbindet, und wobei der Sockel einen Brenner nach einem der Ansprüche 1 bis 11 aufnimmt.

## Claims

1. Catalytic combustion burner comprising a tip (1) made of porous material with a front upper wall (2) and a peripheral side wall (3) jointly delimiting a cavity (4) extending along a principal axis (5) and opening out into a lower opening (6), said tip (1) being intended for covering an upper end of a wick engaged in the cavity (4) in a first direction (7) and suitable for soaking the tip (1) with a combustible composition, said tip (1) also having an upper surface (8) formed from a central zone (9) and a peripheral zone (10) separated from one another by an annular groove (11) hollowed out of the tip (1) in a second direction that is the reverse of the first direction (7), the peripheral wall (3) having an external side surface (12), at least one portion of which is doped by a catalyst, and the central zone (9) of the upper surface (8) being free of any catalyst, **characterised in that** the groove (11) is delimited by side surfaces (13a, 13b) that are inclined relative to the principal axis (5) and that diverge from one another in the first direction (7) and
**in that** the tip (1) includes separate first and second elements (1a, 1b), that are respectively a head (1a) formed from the tip (1) free of any central zone (9) and an insert (1b) forming said central zone (9).

2. Burner according to claim 1, wherein the side surfaces (13a, 13b) of the groove (11) are separated by an α angle (alpha) that lies in the range 30 to 40 degrees.

3. Burners according to either claim 1 or claim 2, wherein the annular groove (11) has a depth (P) that lies in the range 30 to 55%, preferably in the range 40 to 50%, of the height (H) of the tip (1).

4. Burner according to any of claims 1 to 3, wherein the first and second elements (1a, 1b) have at least one contact surface (14, 15) through which they are in contact with each other.

5. Burner according to any of claims 1 to 4, wherein the side surfaces (13a, 13b) of the groove (11), preferably a central side surface (13a) of the groove (11), and/or a front surface (19) of the central zone (9) comprise recesses (17a, 17b).

6. Burners according to claim 5, wherein the recesses (17a, 17b) occupy a surface area that is at least equal to 5%, preferably in the range 10 to 60%, of the side surfaces (13a, 13b) of the groove (11), preferably of the central side surface (13a) of the groove (11), and/or of a front surface (19) of the central zone (9).

7. Burner according to any of claims 1 to 6, wherein the peripheral zone (10) has a front surface (19) that is free of any catalyst.

8. Burner according to any of claims 1 to 7, wherein the tip (1) comprises at least one open channel (16) bringing the cavity (4, 4b) into communication with the atmosphere.

9. Burner according to any of claims 1 to 8, wherein the cavity (4) of the tip (1) extends at the lower opening (6) via a cavity (4') of a sleeve (20) made of porous material and suitable for surrounding the wick.

10. Burner according to any of claims 1 to 9, wherein the outer side surface (12) is inclined over at least one portion of the height thereof relative to the principal axis (5), the inclination being such that the diameter of the tip (1) increases in the first direction (7) .

11. Burner according to any of claims 1 to 10, wherein the peripheral side wall (3) has a support edge (22) capable of being supported by a support device, said support edge (22) comprising at least one hollow zone (23) suitable for defining an air duct at the periphery of the burner.

12. Catalytic combustion device comprising a recipient suitable for receiving a combustible composition, said recipient comprising a neck to which is secured a base comprising a hole bringing the inside of the recipient into communication with the atmosphere, and said base receiving a burner according to any of claims 1 to 11.
